# EUROPEAN PATENT SPECIFICATION

(11) **EP 1 570 789 B1**
(45) Date of publication and mention of the grant of the patent: **14.05.2008**
(21) Application number: 05011573.2
(22) Date of filing: 05.06.1996
(51) Int. Cl.: A61B 17/00, A61B 19/00

(54) **Surgical manipulator for a telerobotic system**
Chirurgischer Manipulator für ein ferngesteuertes Robotersystem
Manipulateur chirurgical pour un systeme telerobotique

(30) Priority: 07.06.1995 US 485587; 07.06.1995 US 487020
(43) Date of publication of application: 07.09.2005
(62) Divisional of application: 96921339.6
(73) Proprietor: SRI International, Menlo Park, California 94025-3493 (US)
(72) Inventor: Jensen, Joel F., Redwood City CA 94061 (US); Hill, John W., Nevada City CA 95959-9295 (US)
(74) Representative: Kazi, Ilya

(56) References cited:
- WO-A-94/03113
- WO-A-95/13023
- US-A- 4 569 131
- US-A- 5 318 586
- US-A- 5 350 355
- US-A- 5 370 134

## Description

### BACKGROUND OF THE INVENTION

This invention relates to surgical manipulators and more particularly to robotically-assisted apparatus for use in surgery.

In standard laparoscopic surgery, a patient's abdomen is insufflated with gas, and trocar sleeves are passed through small (approximately 1/2 inch) incisions to provide entry ports for laparoscopic surgical instruments. The laparoscopic surgical instruments generally include a laparoscope for viewing the surgical field, and working tools such as clamps, graspers, scissors, staplers, and needle holders. The working tools are similar to those used in conventional (open) surgery, except that the working end of each tool is separated from its handle by an approximately 12-inch long extension tube. To perform surgical procedures, the surgeon passes instruments through the trocar sleeves and manipulates them inside the abdomen by sliding them in and out through the sleeves, rotating them in the sleeves, levering (i.e., pivoting) the sleeves in the abdominal wall and actuating end effectors on the distal end of the instruments.

In robotically-assisted and telerobotic surgery (both open and endoscopic procedures), the position of the surgical instruments is controlled by servo motors rather than directly by hand or with fixed clamps. The servo motors follow the motions of a surgeon's hands as he/she manipulates input control devices and views the operation via a displayed image from a location that may be remote from the patient. The servo motors are typically part of an electromechanical device or surgical manipulator that supports and controls the surgical instruments that have been introduced directly into an open surgical site or through trocar sleeves into a body cavity, such as the patient's abdomen. During the operation, the surgical manipulator provides mechanical actuation and control of a variety of surgical instruments, such as tissue graspers, needle drivers, etc, that each perform various functions for the surgeon, i.e., holding or driving a needle, grasping a blood vessel or dissecting tissue.

This new method of performing telesurgery through remote manipulation will create many new challenges. One such challenge is transmitting position, force, and tactile sensations from the surgical instrument back to the surgeon's hands as he/she operates the telerobotic system. Unlike other techniques of remote manipulation, telesurgery can give the surgeon the feeling that he/she is manipulating the surgical instruments directly by hand. For example, when the instrument engages a tissue structure or organ within the patient, the system should be capable of detecting the reaction force against the instrument and transmitting this force to the input control devices. In this manner, the surgeon can see the instrument contacting the tissue structure on the displayed image and directly feel the pressure from this contact on the input control devices. Providing the appropriate feedback, however, can be problematic because of other forces acting on the system, such as friction within the telerobotic mechanisms, gravity and inertial forces acting on the surgical manipulator or forces exerted on a trocar sleeve by the surgical incision.

In addition, to enable effective telesurgery, the manipulator must be highly responsive and must be able to accurately follow even the most rapid hand motions that a surgeon frequently uses in performing surgical procedures. To achieve this rapid and responsive performance, a telerobotic servo system must be designed to have an appropriately high servo bandwidth which requires that the manipulator be designed to have low inertia and to employ drive motors with relatively low ratio gear or pulley couplings.

Another challenge with telesurgery results from the fact that a portion of the electromechanical surgical manipulator will be in direct contact with the surgical instruments, and will also be positioned adjacent the operation site. Accordingly, the surgical manipulator may become contaminated during surgery and is typically disposed of or sterilized between operations. Of course, from a cost perspective, it would be preferable to sterilize the device. However, the servo motors, sensors and electrical connections that are necessary to robotically control the motors typically cannot be sterilized using conventional methods, e.g., steam, heat and pressure or chemicals, because they would be damaged or destroyed in the sterilization process.

Yet another challenge is that different surgical instruments will be attached and detached from the same instrument holder a number of times during an operation. In laparoscopic procedures, for example, the number of entry ports into the patient's abdomen is generally limited during the operation because of space constraints as well as a desire to avoid unnecessary incisions in the patient. Thus, a number of different surgical instruments will typically be introduced through the same trocar sleeve during the operation. Likewise, in open surgery, there is typically not enough room around the surgical site to position more than one or two surgical manipulators, and so the surgeon's assistant will be compelled to frequently remove instruments from the holder and exchange them with other surgical tools.

What is needed, therefore, is a system and method for holding and manipulating surgical instruments by remote control and for releasably coupling a surgical instrument to an instrument holder. The apparatus should be configured for easy sterilization so that it can be reused after it has been contaminated during an operation. The apparatus should be further capable of providing the surgeon with the appropriate feedback from forces transmitted to and from the surgical instrument during the telerobotic operation and it should be configured to compensate for gravitational forces acting on the apparatus so that these forces are not felt by the surgeon. In addition, the apparatus must be highly responsive and must be able to accurately follow even the most rapid hand motions that a surgeon frequently uses in performing surgical procedures. It would further be desirable to provide a system configured to quickly and easily engage and disengage the instrument from the holder to minimize the instrument exchange time during endoscopic surgery.

US 4,569,131 describes a manually operated pincer-like tool for surgical applications; when pushing forces are exert on a rod located inside a hollow tube, the pincer is closed. WO 95/13023 describes a similar device, this time also featuring an impinging device to enable the rod to be locked in intermediate positions. Likewise, US 5,350,355 details a grasping or cutting tool that is activated by a linkage inside a hollow tube; in this case the linkage is activated by a linear solenoid.

### SUMMARY OF THE INVENTION

According to a first aspect of the present invention, there is provided a surgical instrument comprising: an elongate shaft having a proximal portion, a distal portion, an inner lumen, and at least one axially extending slot; an end effector disposed adjacent the distal portion of the elongate shaft; and an actuator assembly comprising a linkage disposed within the inner lumen and at least one moveable actuator pin laterally extending from the linkage and extending through the axially extending slot to releasably couple the actuator assembly to a driver of a robotic surgical instrument holder.

According to a second aspect of the present invention, there is provided a method for controlling a surgical instrument comprising: providing a surgical instrument removably coupleable to an actuating mechanism, said instrument comprising proximal and distal portions, said proximal portion comprising at least one movable actuator pin engageable with an actuating body on said actuating mechanism, said instrument including at least one distal joint coupled to an end effector member, said actuated body coupled to said distal joint by at least one elongate element, said elongate element being housed in the inner lumen of a shaft portion of said instrument and extending between said proximal and distal portions, said shaft portion having at least one axially extending slot, said at least one movable pin laterally extending from said elongate element and extending through the axially extending slot; coupling said instrument to said actuating mechanism by engaging said at least one movable actuator pin with said actuating mechanism; and controlling the operation of said actuating mechanism from a remote location so that said actuating body on said actuating mechanism drives said at least one movable actuator pin of the instrument, thereby causing the at least one distal joint of the instrument to move.

There is provided herein, an apparatus for releasably holding and manipulating a surgical instrument during conventional open surgery or endoscopic procedures, such as laparoscopy. The apparatus includes a manipulator assembly having an instrument holder removably coupled to a drive assembly so that the holder can be sterilized. The assembly further includes a force sensing element mounted distal to the holder and the drive assembly for detecting forces exerted on the surgical instrument and providing feedback to the surgeon. The invention is also directed to a system and method for releasably holding a surgical instrument. The system includes an instrument support for automatically locking mounting pins on the instrument within locking slots on the instrument holder to releasably couple the instrument to the holder. With a twist-lock motion, the surgeon can rapidly engage and disengage various instruments from the holder during a surgical procedure, such as open surgery, laparoscopy or thoracoscopy.

In one configuration, the apparatus comprises a support base fixable by means of various passive or power driven positioning devices to a surface, such as an operating table, and an instrument holder movably mounted on the base. The instrument holder comprises a body and an instrument support movably coupled to the body and having an interface engageable with the surgical instrument to releasably mount the instrument to the instrument holder. A drive assembly is operatively coupled to the instrument holder for providing the instrument with at least two degrees of freedom. The drive assembly includes a first drive for moving the instrument support and a second drive for moving the instrument holder relative to the support base. The apparatus includes means for removably coupling the instrument holder from the base and the drive assembly so that the holder can be separated from the rest of the device and sterilized after a surgical procedure.

In a specific configuration, the support base includes a frame with distal and proximal support members and a pair of shafts rotatably mounted within the support members. The instrument holder is slidably mounted on the support shafts for axial movement of the instrument. In addition, the shafts are each coupled to a drive motor for providing the instrument with second and third degrees of freedom, e.g., rotation and end effector actuation. The drive motors are coupled to the proximal support member so that they will not be contaminated during surgery. The rotatable shafts can be removed_by sliding them upward and out of engagement with their lower bearings and the instrument holder so that the instrument holder can be easily removed from the support base for sterilization. The lower portion of the support base (including the distal support member) may also be sterilized to decontaminate those parts that have contacted the instrument holder. In this manner, the surgical manipulator can be easily sterilized after a surgical procedure without damaging the servo motors or the electrical connections required for the telerobotic system.

The support base further comprises a sleeve, such as a cannula or trocar sleeve, mounted on the distal support member. The sleeve has an axial passage for receiving the instrument therethrough and a force sensing element mounted within the axial passage near the distal end of the sleeve. The force sensing element is configured to detect lateral forces exerted on the element by the distal portion of the instrument during surgery. Since the force sensing element is mounted distal to the remainder of the apparatus, it is undisturbed by forces that may be exerted on the cannula by the surgical incision or by gravity and inertial forces that act on the instrument holder. When supported by a positioning device, the surgical manipulator can be used with an inclinometer to determine the true orientation of the instrument holder with respect to the direction of the local gravitational field. Use of the inclinometer and force sensors with the manipulator facilitates the design of a telerobotic system in which the surgeon will directly sense the forces acting against the end of the instrument, unaffected by extraneous forces acting on the telerobotic mechanism. In other words, the surgeon will feel as if his/her hands are holding the instrument at the point in which the instrument contacts the force sensing element.

The invention is particularly useful for holding and manipulating a surgical instrument having an end effector, such as a pair of jaws, coupled to the distal end of the instrument shaft. To that end, the instrument holder further includes an actuator driver having an interface engageable with an end effector actuator on the instrument. The actuator driver includes a coupling that connects the driver to the drive assembly for axially moving a portion of the driver relative to the support base, thereby actuating the end effector of the instrument. In a preferred configuration, the coupling is a concentric helical actuator that translates rotation from a drive motor into axial movement of the end effector actuator. Because of the symmetrical design of the helical actuator, the actuation force applied by the drive motor will not generate any effective side loads on the instrument, which avoids frictional coupling with other degrees of freedom such as axial movement and rotation of the instrument.

In another configuration, the instrument comprises an elongate shaft with proximal and distal ends and a mounting means having a protrusion extending radially from the shaft between the proximal and distal ends. An instrument holder comprises a support having a body with an axial passage for receiving the instrument shaft and a first hole in communication with the axial passage for receiving the protrusion. A second hole is cut into the body transversely to and in communication with the first hole so that the protrusion can be rotated within the second hole. To prevent the instrument from being accidently twisted and thereby disengaged from the instrument holder during surgery, the holder further includes a locking means coupled to the body for automatically locking the protrusion within the second hole thereby releasably locking the instrument to the instrument holder.

In a preferred configuration, the protrusion of the mounting means comprises a pair of opposing arms, such as mounting pins, extending outward from the instrument shaft. The first hole is an axially extending slot for receiving the mounting pins and the second hole is a perpendicular locking slot having a first portion aligned with the axial slot and a second portion extending circumferentially around the body of the instrument support. With this configuration, the mounting pins can be slid through the axial slot and rotated into the locking slot to attach the instrument to the holder. The instrument can be removed by performing the same two steps in reverse order. With this twist-lock motion, the surgeon can rapidly engage and disengage various instruments from the instrument holder during a surgical procedure.

The locking means preferably comprises a releasable latch assembly for locking the mounting pins to the instrument holder. The latch assembly includes a spring-loaded plunger coupled to a latch that normally locks the instrument in place by capturing the mounting pin in the locking slot. The plunger has a button extending outward from the instrument holder for moving the latch away from the locking slot. The button can be depressed manually or automatically to release the mounting pins and allow instrument exchange when the instrument is easily accessible to the surgeon.

The invention is particularly useful for releasably holding an endoscopic instrument configured for introduction through a small percutaneous penetration into a body cavity, e.g., the abdominal or thoracic cavity. To that end, the instrument preferably includes an end effector, such as a pair of jaws, coupled to the distal end for engaging a tissue structure within the body cavity. To actuate the end effector, the instrument has a second pair of arms, such as actuator pins, laterally extending from the shaft and operatively coupled to the end effector. Preferably, the actuator pins are axially displaceable with respect to the shaft to actuate the end effector (e.g., open and close the jaws). The instrument holder further includes an actuator driver releasably coupled to the actuator arms and to an external driver for actuating the end effector. The actuator driver preferably includes a twist-lock interface having transverse slots similar to that described for the instrument support so that the instrument can be simultaneously engaged or disengaged from both the instrument support and the actuator driver.

Other features and advantages of the invention will appear from the following description in which the preferred embodiment has been set forth in detail in conjunction with the accompanying drawings.

### BRIEF DESCRIPTION OF THE DRAWINGS

Fig. 1 is a partial sectional elevational view of a robotic endoscopic surgical instrument mounted to a manipulator assembly according to the present invention;
Fig. 1A is a partial sectional elevational view of the manipulator assembly of Fig. 1 illustrating the removal of an instrument holder from the rest of the assembly;
Figs. 2A and 2B are enlarged side and front cross-sectional views, respectively, of the surgical instrument of Fig. 1;
Figs. 3A and 3B are perspective views of an instrument support and an actuator pin catch, respectively, for releasably mounting the surgical instrument to the manipulator assembly;
Fig. 4 is a front elevational view of the surgical instrument mounted within the instrument support and actuator pin catch of Figs. 3A and 3B;
Fig. 5 is a front elevational view of an actuator driver for providing axial movement of the actuator pin catch of Fig. 3B;
Figs. 6A and 6B are enlarged cross-sectional views of an actuator carriage assembly and a helical actuator of the actuator driver of Fig. 5;
Fig. 7 is an enlarged detail of a portion of the frame of the manipulator assembly of Fig. 1 illustrating a coupling mechanism for removing the shafts from the frame;
Fig. 8 is a partial cross-sectional view of the instrument support of Fig. 3A illustrating a locking mechanism for a twist lock interface according to the present invention; and
Fig. 9 is an elevational view of a remote center positioner for holding the manipulator assembly of Fig. 1.

### DESCRIPTION OF THE PREFERRED EMBODIMENTS

Referring to the drawings in detail, wherein like numerals indicate like elements, a manipulator assembly 2 is illustrated according to the principles of the invention. Manipulator assembly 2 generally includes an instrument holder 4 removably mounted to a base 6 and a drive assembly 7 for manipulating a surgical instrument 14 releasably coupled to instrument holder 4.

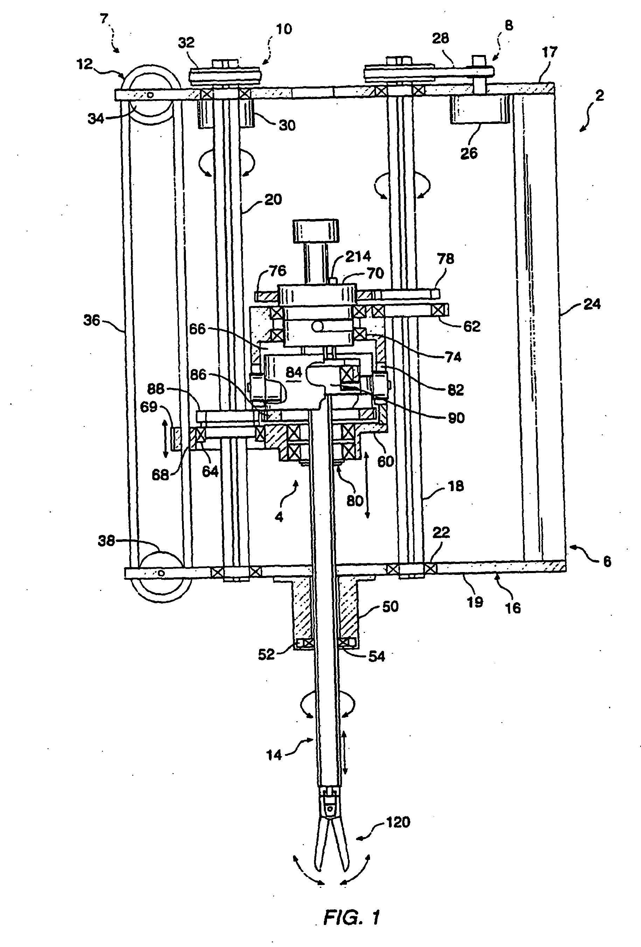

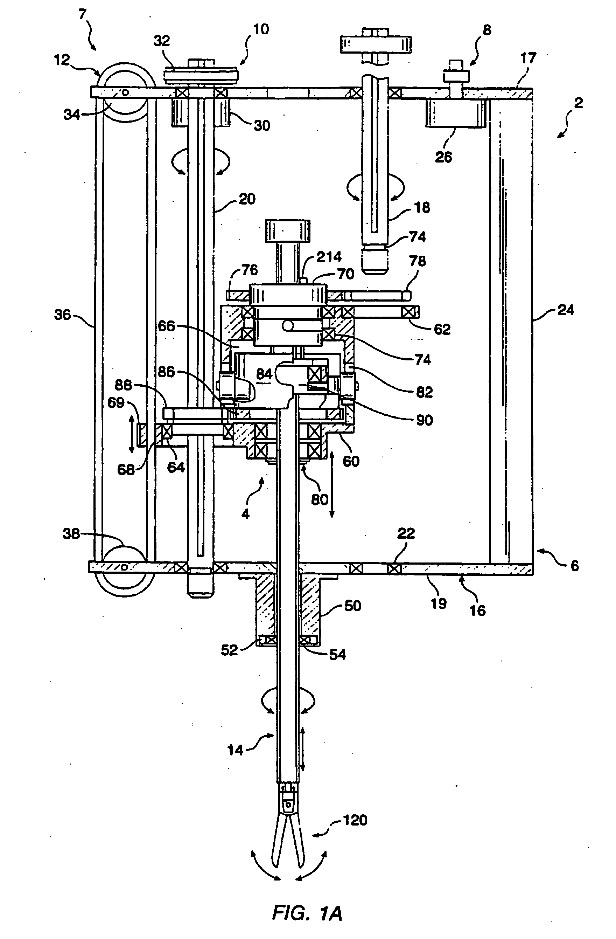

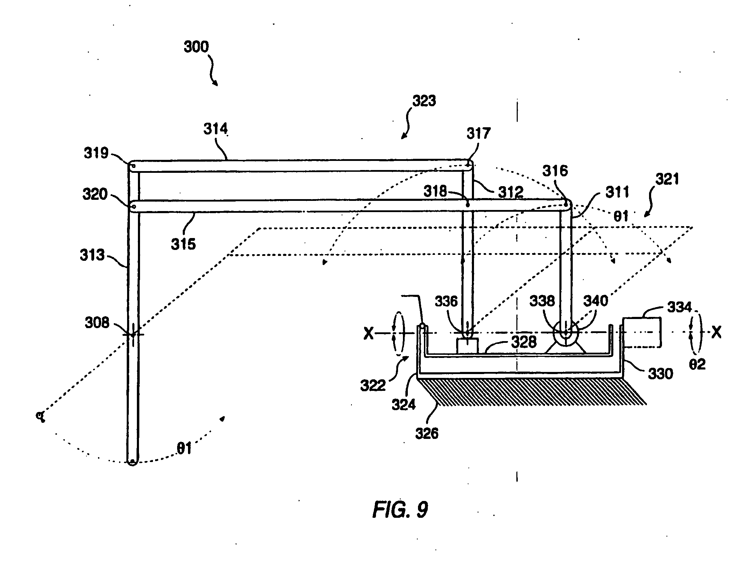

Referring to Fig. 1, base 6 comprises a frame 16 having proximal and distal elongate support members 17, 19 and first and second ball-spline shafts 18, 20 rotatably coupled to support members 17, 19 via bearings 22. Frame 16 further includes a support bracket 24 for attaching manipulator assembly 2 to a remote center positioner 300, as discussed in more detail below (see Fig. 9). Drive assembly 7 comprises first, second and third drives 8, 10, 12, which are mounted to frame 16 and configured to provide three degrees of freedom to surgical instrument 14. In the preferred embodiment, first drive 8 rotates instrument 14 around its own axis, second drive 10 actuates an end effector 120 on the distal end of instrument 14 and third drive 12 axially displaces instrument 14 with respect to frame 16. Of course, it will be readily recognized by those skilled in the art that other configurations are possible. For example, assembly 2 may include additional drives for providing additional degrees of freedom to surgical instrument 14, such as rotation and flexion of an instrument wrist.

First drive 8 comprises a rotation drive motor 26 fixed to frame 16 and coupled to first shaft 18 by a drive belt 28 for rotating first shaft 18 with respect to frame 16. Second drive 10 comprises a gripper drive motor 30 fixed to frame 16 and coupled to second shaft 20 by a drive belt 32 for rotating second shaft 20 with respect to frame 16. Third drive 12 comprises a vertical drive motor 34 coupled to instrument holder 4 via a drive belt 36 and two pulleys 38 for axially displacing instrument holder 4 with respect to frame 16. Drive motors 26, 30, 34 are preferably coupled to a controller mechanism via servo-control electronics (not shown) to form a telerobotic system for operating surgical instrument 14 by remote control. The drive motors follow the motions of a surgeon's hands as he/she manipulates input control devices at a location that may be remote from the patient. A suitable telerobotic system for controlling the drive motors is described in commonly assigned co-pending application Serial No. 08/823,932 filed 1/21/92 TELEOPERATOR SYSTEM AND METHOD WITH TELEPRESENCE, which is incorporated herein by reference.

The above described telerobotic servo system preferably has a servo bandwidth with a 3 dB cut off frequency of at least 10 hz so that the system can quickly and' accurately respond to the rapid hand motions used by the surgeon. To operate effectively with this system, instrument holder 4 has a relatively low inertia and drive motors 26, 30, 34 have relatively low ratio gear or pulley couplings.

In a specific embodiment, surgical instrument 14 is an endoscopic instrument configured for introduction through a percutaneous penetration into a body cavity, such as the abdominal or thoracic cavity. In this embodiment, manipulator assembly 2 supports a cannula 50 on distal support member 19 of frame 16 for placement in the entry incision during an endoscopic surgical procedure (note that cannula 50 is illustrated schematically in Fig. 1 and will typically be much longer). Cannula 50 is preferably a conventional gas sealing trocar sleeve adapted for laparoscopic surgery, such as colon resection and Nissen fundoplication.

As shown in Fig. 1, cannula 50 preferably includes a force sensing element 52, such as a strain gauge or force-sensing resistor, mounted to an annular bearing 54 within cannula 50. Bearing 54 supports instrument 14 during surgery, allowing the instrument to rotate and move axially through the central bore of bearing 54. Bearing 54 transmits lateral forces exerted by the instrument 14 to force sensing element 52, which is operably connected to the controller mechanism for transmitting these forces to the input control devices (not shown) held by the surgeon in the telerobotic system. In this manner, forces acting on instrument 14 can be detected without disturbances from forces acting on cannula 50, such as the tissue surrounding the surgical incision, or by gravity and inertial forces acting on manipulator assembly 2. This facilitates the use of manipulator assembly in a robotic system because the surgeon will directly sense the forces acting against the end of instrument 14. Of course, the gravitational forces acting on the distal end of instrument 14 will also be detected by force sensing element 52. However, these forces would also be sensed by the surgeon during direct manipulation of the instrument.

As shown in Fig. 1, instrument holder 4 comprises a chassis 60 mounted on shafts 18, 20 via ball-spline bearings 62, 64 so that chassis 60 may move axially with respect to shafts 18, 20, but is prevented from rotating with shafts 18, 20. Chassis 60 is preferably constructed of a material that will withstand exposure to high temperature sterilization processes, such as stainless steel, so that chassis 60 can be sterilized after a surgical procedure. Chassis 60 includes a central cavity 66 for receiving surgical instrument 14 and an arm 68 laterally extending from chassis 60. Arm 68 is fixed to drive belt 36 so that rotation of drive belt 36 moves instrument holder 4 in the axial direction along shafts 18, 20.

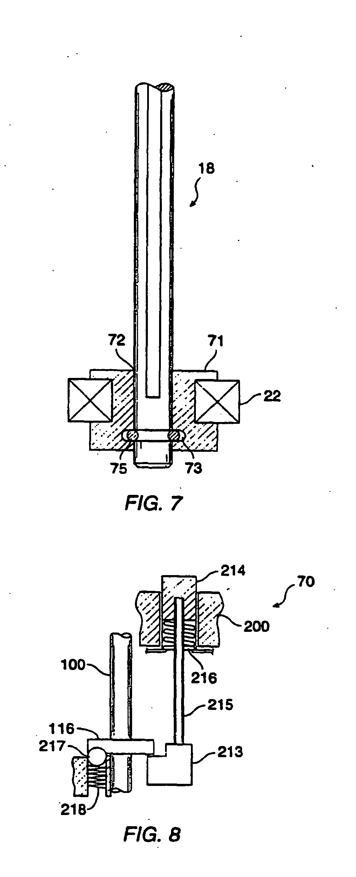

Instrument holder 4 is removably coupled to base 6 and the drive motors so that the entire holder 4 can be removed and sterilized by conventional methods, such as steam, heat and pressure, chemicals, etc. In the preferred configuration, arm 68 includes a toggle switch 69 that can be rotated to release arm 68 from drive belt 36 (Fig. 1). In addition, shafts 18, 20 are removably coupled to bearings 22 so that the shafts can be axially withdrawn from support members 17, 19 of frame 16, as shown in Fig. 1A. To this end, the distal bearings 22 preferably include a coupling mechanism for allowing the removal of shafts 18, 20. As shown in Fig. 7, distal support member 19 includes a support collar 71 within each distal bearing 22 having an inner bore 72 for passage of one of the shafts 18, 20. Each support collar 71 has an internal groove 73 and shafts 18, 20 each have an annular groove 74 (see Fig. 1A) near their lower ends that is aligned with internal grooves 73 when the shafts are suitably mounted within frame 16 (Fig. 1). A spring clip 75 is positioned within each internal groove 73 to hold each shaft 18, 20 within the respective support collar 71. Spring clip 74 has a discontinuity (not shown) to allow removal of shafts 18, 20 upon the application of a threshold axial force on the shafts.

To remove instrument holder 4 from base 6, the operator rotates toggle switch 69 to release arm 68 from drive belt 36 and removes drive belts 28, 32 from drives 8, 10. As shown in Fig. 1A, the operator holds instrument holder 4 and pulls shafts 18, 20 upwards, providing enough force to release spring clips 75. Shafts 18, 20 will disengage from distal bearings 22 and slide through ball-spline bearings 62, 64 so that instrument holder 4 is disconnected from base 6. It should be understood that the invention is not limited to the above described means for removably coupling instrument holder 4 to base 6 and drive assembly 7. For example, distal support member 19 may be removably coupled to the rest of frame 16 so that the surgeon simply removes member 19 and slides holder down and off shafts 18, 20. Proximal support member 17 may be removably coupled to frame 16 in a similar manner. Alternatively, the drive motors may be housed in a separate servo-box (not shown) that is removably attached to base 6. In this configuration, the servo-box would be removed from base 6 so that the entire base 6, together with holder 4, can be sterilized.

The lower portion of base 6 (including distal support member 19) may also be sterilized to decontaminate those parts that come into contact with holder 4 or instrument 14 (e.g., by dipping the lower portion of base 6 into a sterilizing bath). To facilitate this type of sterilization, shafts 18, 20 will preferably be somewhat longer than shown in Fig. 1 so that the upper portion of base 6, including drive assembly 7, is disposed sufficiently away from holder 4 and instrument 14. In this manner, the surgical manipulator can be easily sterilized after a surgical procedure without damaging the drive motors or the electrical connections required for the telerobotic system.

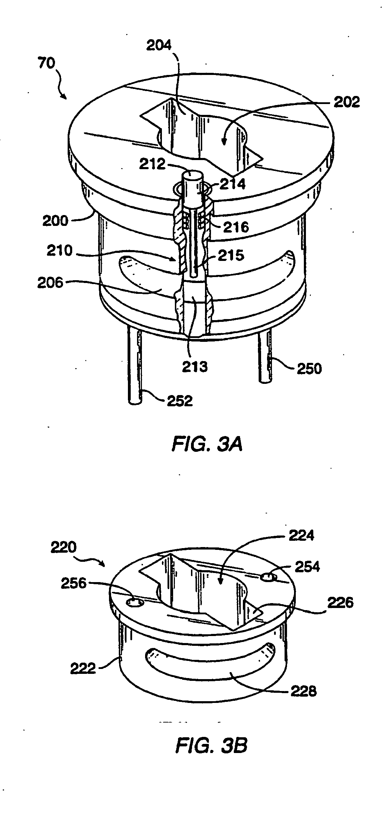

Instrument holder 4 further includes an instrument support 70 (see detail in Fig. 3A), for releasably coupling surgical instrument 14 to the manipulator assembly. Instrument support 70 is rotatably mounted within chassis 60 via mounting bearings 74 so that support 70 and the instrument can be rotated therein. As shown in Fig. 1, support 70 is circumscribed by an annular ring gear 76 having teeth that mesh with the teeth of a drive gear 78 mounted to first shaft 18. Drive gear 78 is configured around first shaft 18 such that it will rotate with first shaft 18, thereby rotating instrument support 70 and the surgical instrument therewith. Drive gear 78 is also configured to move axially with respect to first shaft 18 to allow axial movement of instrument holder 4 with respect to frame 16.

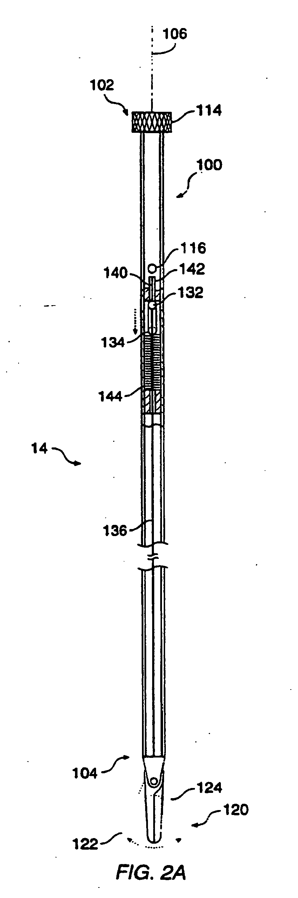

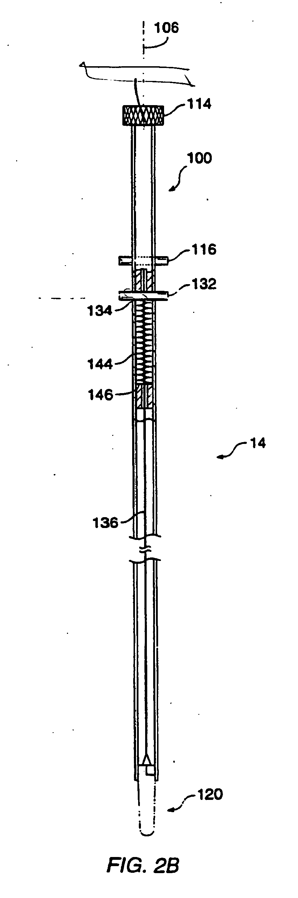

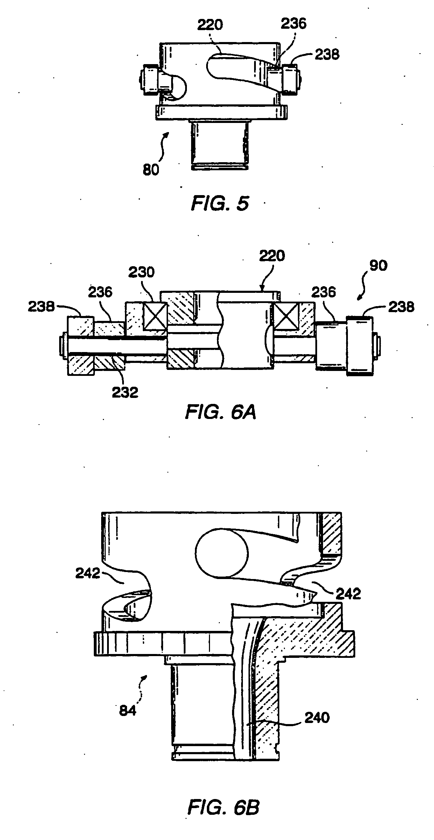

Instrument holder 4 further includes an actuator driver 80 (see detail in Fig. 5) movably mounted within axial guide slots 82 on either side of chassis 60. Actuator driver 80 comprises a helical actuator 84 (see detail in Fig. 6B) having a ring gear 86 that meshes with a gripper drive gear 88 mounted to second shaft 20. Rotation of second shaft 20 causes rotation of gripper drive gear 88, thereby rotating ring gear 86 and helical actuator 84 within chassis 60. Actuator driver 80 further includes an actuator carriage assembly 90 (see detail in Fig. 6A) for releasably coupling an end effector actuator of surgical instrument 14 to instrument holder 4 (see Fig. 2). Carriage assembly 90 is mounted within helical actuator 84 and chassis 60 such that rotation of helical actuator 84 causes a corresponding axial movement of carriage assembly 90 with respect to chassis 60, as discussed in greater detail below.

Figs. 2A and 2B illustrate a specific embodiment of an endoscopic surgical instrument 14 capable of being operated by a motorized manipulator, such as manipulator assembly 2, for telerobotic surgery. Surgical instrument 14 can be a variety of conventional endoscopic instruments adapted for delivery through a percutaneous penetration into a body cavity, such as tissue graspers, needle drivers, microscissors, electrocautery dissectors, etc. In the preferred embodiment, instrument 14 is a tissue grasper comprising a shaft 100 having a proximal end 102, a distal end 104 and a longitudinal axis 106 therebetween. A knurled handle 114 is attached to proximal end 102 of shaft 100 to facilitate manipulation of instrument 14.

Shaft 100 is preferably a stainless steel tube having an outer diameter in the range of 2-10 mm, usually 4-8 mm, so as to fit within a cannula having an internal diameter in the range of 2-15 mm. Shaft 100 can also be introduced directly through a percutaneous incision in the patient. Shaft 100 has a length selected to reach a target site in a body cavity, such as the abdomen, and to extend sufficiently out of the body cavity to facilitate easy manipulation of surgical instrument 14. Thus, shaft 100 should be at least between 10 cm and 40 cm and is preferably between 17 cm and 30 cm. It should be noted that although shaft 100 is shown as having a circular cross-sectional shape in the drawings, shaft 100 could alternatively have a rectangular, triangular, oval or channel cross-sectional shape.

In a specific configuration, shaft 100 includes a mounting means for releasably coupling surgical instrument 14 to instrument support 70 and first drive 8 of manipulator assembly 2. In the preferred embodiment, mounting means comprises a pair of opposed mounting pins 116 extending laterally outward from shaft 100. Mounting pins 116 are rigidly connected to shaft 100 and are adapted for engaging a twist-lock interface on instrument support 70, as discussed in detail below. It should be understood that the invention is not limited to a pair of opposing pins and mounting means can include a single mounting pin or a plurality of pins extending circumferentially around shaft. Alternatively, pins 116 may have a variety of other shapes, such as spherical or annular, if desired.

Instrument 14 includes an end effector 120 extending from distal end 104 for engaging a tissue structure on the patient, such as the abdomen during laparoscopic surgery. In the preferred embodiment, end effector 120 comprises a pair of jaws 122, 124 that are movable between open and closed positions for grasping a blood vessel, holding a suture, etc. Jaws 122, 124 preferably have transverse grooves or other textural features (not shown) on opposing surfaces to facilitate gripping of the tissue structure. To avoid the possibility of damaging the tissue to which jaws 122, 124 are applied, the jaws may also include atraumatic means (not shown), such as elastomeric sleeves made of rubber, foam or surgical gauze wrapped around jaws 122, 124.

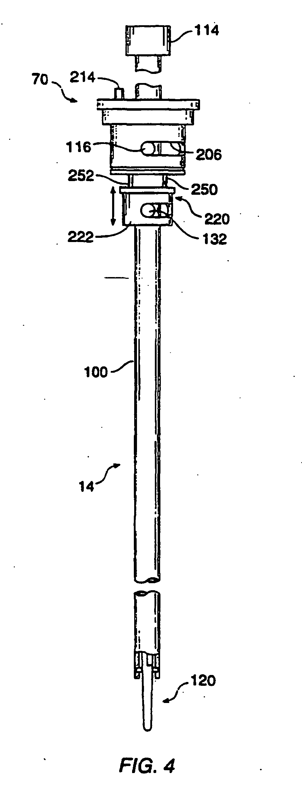

To move jaws 122, 124 between the open and closed positions, instrument 14 includes an end effector actuator releasably coupled to actuator driver 80 and second drive 10 of manipulation assembly 2 (see Fig. 4). In the preferred embodiment, end effector actuator comprises a pair of opposed actuator pins 132 laterally protruding from axially extending slots 134 in shaft 100. Actuator pins 132 are coupled to an elongate rod 136 slidably disposed within an inner lumen 138 of shaft 100. Actuator pins 132 are slidable within slots 134 so that rod 136 is axially movable with respect to shaft 100 and mounting pins 116 to open and close jaws 122, 124, as is conventional in the art. Elongate rod 136 has a proximal portion 140 that is disposed within an inner lumen 142 within shaft 100 to prevent actuator pins 132 from moving in the laterally direction and to ensure that rod 136 remains generally centered within shaft 100 during a surgical procedure.

Jaws 122, 124 are preferably biased into the closed positioned by an annular compression spring 144 positioned within shaft 100 between actuator pins 132 and an annular disc 146 fixed to the inside surface of shaft 100. During endoscopic procedures, this allows the surgical team to introduce jaws 122, 124 through cannula 50 (or any other type of percutaneous penetration) and into the body cavity without getting stuck within cannula 50 or damaging surrounding tissue.

Figs. 3A, 3B and 4 illustrate a twist lock mechanism for releasably connecting surgical instrument 14 to manipulator assembly 2 so that different instruments may be rapidly changed during an endoscopic surgical procedure. As shown in Fig. 3A, instrument support 70 comprises an annular collar 200 defining a central bore 202 for receiving shaft 100 of surgical instrument 14. Collar 200 further defines an axially extending slot 204 in communication with bore 202 and sized to allow mounting and actuator pins 116, 132 of instrument 14 to slide therethrough (see Fig. 4). Two locking slots 206 are cut into annular collar 200 at a transverse angle, preferably about 90°, to axially extending slot 204 (note that only one of the locking slots are shown in Fig. 3A). Locking slots 206 intersect slot 204 near the center of annular collar 200 and extend circumferentially around bore 202, preferably about 90°, to allow rotation of both mounting pins 116 therethrough, as discussed below.

As shown in Figs. 3A and 8, instrument support 70 further comprises means for locking mounting pins 116 into locking slots 206 so that the instrument cannot be accidently twisted and thereby disengaged from instrument support 70 during surgery. Preferably, the locking means comprises a latch assembly having a plunger 210 slidably disposed within a hole 212 in collar 200, as shown in Fig. 3A. Plunger 210 comprises an L-shaped latch 213 coupled to a release button 214 by a rod 215 extending through hole 212. Plunger 210 is movable between a first position, where latch 213 is not disposed within locking slots 206 so that mounting pins 116 are free to rotate therethrough, and a second position, where latch 213 is at least partially disposed within one of the locking slots 206 so as to prevent rotation of mounting pins 116. Latch 213 is preferably biased into the second or locked position by a compression spring 216.

Button 214 is disposed on the upper surface of support 70 for manual actuation by the surgeon or automatic actuation by base 6. Preferably, when instrument holder 4 is moved to its most proximal position (see Fig. 1), proximal support member 17 of frame 16 depresses release switch 214 to move latch 213 into the first or open position. With this configuration, instruments can be exchanged only when the instrument holder 4 is in the most proximal position, where shaft 100 of instrument 14 is easily accessible. In addition, this prevents the accidental release of the instrument when its distal end has penetrated cannula 50 and is disposed within the body cavity.

The intersecting axial and locking slots 204, 206 form an interface for releasably coupling mounting pins 116 of surgical instrument 14 to instrument holder 4. To insert instrument 14, the surgeon aligns mounting pins 116 with axial slot 204 and slides the instrument through bore 202 of annular collar 200 until mounting pins 116 are aligned with locking slots 206, as shown in Fig. 4. The instrument is then rotated a sufficient distance, preferably about a 1/4 turn, through locking slots 206 so that the pins are no longer aligned with axial slot 204. When instrument 14 is moved distally, switch 214 is released (Fig. 1) and latch 213 moves into locking slots 206 to prevent mounting pins 116 from rotating back into alignment with axial slot 204 so that instrument 14. is secured to instrument support 70. It should be noted that a single mounting pin may be utilized with the above described configuration to lock the surgical instrument to the support. However, two opposing pins are preferred because this configuration reduces torsional forces on the inner surface of locking slots 206.

As shown in Fig. 8, the locking means preferably includes a ball detent 217 disposed within collar 200. Ball detent 217 is biased upward into one of the locking slots 206 by a spring 218. Ball detent 217 serves to temporarily capture mounting pins 116 in a position rotated about 90° from alignment with axial slot 204. This ensures that the mounting pins will be completely rotated into the proper position (i.e., out of the way of latch 213) when instrument 14 is twisted into instrument holder. Otherwise, when switch 214 is released, latch 213 could become engaged with mounting pins 216 so that the latch is unable to move completely into the locked position, thereby potentially causing the accidental release of instrument 14 during surgery.

As shown in Figs. 3B, 4 and 5, actuator driver 80 of instrument holder 4 further comprises an actuator pin catch 220 for releasably holding and moving actuator pins 132 of instrument 14. Actuator pin catch 220 is constructed similarly to instrument support 70 (Fig. 3A), comprising an annular collar 222 that defines a bore 224 for receiving shaft 100 and an axially extending slot 226 for receiving actuator pins 132. A locking slot 228 is cut into actuator pin catch 220 at a 90° angle so that actuator pins can be rotated into the lock slot to couple actuator pins 132 to actuator driver 66, as discussed above in reference to the mounting pins. It should be noted that slot 226 need not extend completely through collar 222 since actuator pins 132 are located distally of mounting pins 116 (the instrument is preferably inserted jaws first). Of course, actuator and mounting pins 132, 116 may be reversed so that the mounting pins are distal to the actuator pins, if desired.

Referring to Fig. 6A, actuator pin catch 220 is rotatably mounted on a ball bearing 230 in actuator carriage assembly 90. Bearing 230 allows the pin catch 220 to rotate freely in carriage assembly 90 while preventing relative axial motion. Therefore, when instrument 14 is rotated by first drive 8, actuator pins 132 will rotate within carriage assembly 90. Carriage assembly 90 further comprises two sets of axles 232 for rotatably supporting a pair of inner rollers 236 and a pair of outer rollers 238. As shown in Fig. 1, outer rollers 238 are slidably disposed within axial guide slots 82 of chassis 60 to prevent rotation of carriage assembly 90 with respect to chassis 60. Inner and outer rollers 236, 238 cooperate with helical actuator 84 and chassis 60 of instrument holder 4 to move axially with respect to the holder, thereby axially moving pin catch 220 and actuator pins 132 therewith relative to shaft 100 of instrument 14 (which actuates jaws 122, 124, as discussed above).

As shown in Fig. 6B, helical actuator 84 includes a central bore 240 for receiving carriage assembly 90 and surgical instrument 14 and two opposing helical tracks 242, 244 each extending circumferentially around helical actuator 84 (preferably slightly less than 180°) for receiving inner rollers 236 of carriage assembly 90, as shown in Fig. 5. With outer rollers 238 constrained in axial guide slots 82 of chassis 60, rotation of helical actuator 84 causes carriage assembly 90 (and actuator pin catch 220) to move up or down, depending on the sense of the rotation. Because of the symmetrical design of helical actuator 84, the actuation force applied by second driver 10 will not generate any effective side loads on instrument 14, which avoids frictional coupling with other degrees of freedom such as axial (third driver 12) and rotation (first driver 8). In the preferred embodiment, helical tracks 242, 244 have a pitch selected such that the mechanism can be easily back-driven, allowing grip forces to be sensed in a position-servoed teleoperation system.

As shown in Figs. 3A and 3B, instrument holder 4 further includes a pair of axial guide pins 250, 252 fixed to instrument support 70. Actuator pin catch 220 has a pair of openings 254, 256 for receiving guide pins 250, 252. Guide pins 250, 252 prevent relative rotation between pin catch 220 and support 70 (so that actuator and mounting pins 116, 132 can both rotate with the instrument) and allow axial movement relative to each other (so that end effector 120 can be actuated by axial movement of actuator pins 132).

Fig. 9 is an elevational view of a remote center positioner 300 which can be used to support manipulator assembly 2 above the patient (note that support manipulator 2 is not shown in Fig. 8). Remote center positioner 300 provides two degrees of freedom for positioning manipulator assembly 2, constraining it to rotate about a point 308 coincident with the entry incision. Preferably, point 308 will be approximately the center of bearing 54 in cannula 50 (Fig. 1). A more complete description of remote center positioner 300 is described in commonly assigned patent Serial No. 6406472 filed 5/14/93 REMOTE CENTER POSITIONER.

A first linkage means is indicated generally by the numeral 321 and a second linkage in the form of a parallelogram is indicated by the numeral 323. The first linkage means is pivotally mounted on a base plate for rotation about an x-x axis. The second linkage means is pivotally connected to the first linkage means and is adapted to move in a plane parallel to the first linkage. Five link members (including extensions thereof), 311, 312, 313, 314, and 315 are connected together with pivot joints 316-320. A portion of element 313 extends beyond pivot 320 of the parallelogram linkage. The parallelogram linkage has an operating end at link member 313 and a driving end at link member 312. The elongated element 313. may, as desired later, carry a surgical instrument or other device, such as support bracket 24 of manipulator assembly 2. The pivot joints allow relative motion of the link members only in the plane containing them.

A parallelogram linkage is formed by corresponding link members 314, 315 and link members 312 and 313. The portions of link members 314 and 315 of the parallelogram are of equal length as are the portions of members 312 and 313 of the parallelogram. These members are.connected together in a parallelogram for relative movement only in the plane formed by the members. A rotatable joint generally indicated by the numeral 322 is connected to a suitable base 324. The rotatable joint 322 is mounted on a base plate 326 adapted to be fixedly mounted to the base support means 324. A pivot plate 328 is pivotally mounted to base plate 326 by suitable means at, such as, pivots 330, 332. Thus pivot plate 328 may be rotated about axis x-x through a desired angle θ2. This may be accomplished manually or by a suitable pivot drive motor 334.

A first linkage is pivotally mounted on the pivot plate 328 of the rotatable joint 322. The linkage elements 311, 312 and the link members are relatively stiff or inflexible so that they may adequately support an instrument used in surgical operations. Rods made of aluminum or other metal are useful as such links. The linkage elements 311 and 312 are pivotally mounted on base plate 328 for rotation with respect to the rotatable joint by pivots 336 and 338. At least one of the pivots 336, 338 is positioned so that its axis of rotation is normal to and intersects the x-x axis. Movement may occur manually or may occur using a linkage drive motor 340. The first linkage is also shaped in the form of a parallelogram formed by linkage elements 311, and 312; the portion of link member 315 connected thereto by pivots 316, 318; and base plate 328. One of the link members 315 is thus utilized in both the first 321 and second 323 linkage means. Linkage element 312 also forms a common link of both the first linkage means 321 and the second linkage means 323. In accordance with the invention, a remote center of spherical rotation 308 is provided by the above described embodiment of apparatus when the linkage element 311 is rotated and/or when pivot plate 328 is rotated about axis x-x. Thus, the end of element 313 can be moved through desired angles θ1 and θ2 or rotated about its own axis while the remote center of rotation remains at the same location.

Fig. 9 also shows an inclinometer 350 attached to the base of remote center positioner 300. The remote center positioner may be mounted at an arbitrary orientation with respect to vertical depending on the particular surgery to be performed, and inclinometer 350 can be used to measure this orientation. The measured orientation can be used to calculate and implement servo control signals necessary to control the telerobotic system so as to prevent gravitational forces acting on the system mechanisms from being felt by the surgeon.

Variations and changes may be made by others without departing from the scope of the present claims. For example, it should be understood that the present invention is not limited to endoscopic surgery. In fact, instrument holder 4, along with a telerobotic control mechanism, would be particularly useful during open surgical procedures, allowing a surgeon to perform an operation from a remote location, such as a different room or a completely different hospital.

## Claims

1. A surgical instrument (14) comprising:
an elongate shaft (100) having a proximal portion, a distal portion, an inner lumen, and at least one axially extending slot (134);
an end effector (120) disposed adjacent the distal portion of the elongate shaft (100); and
an actuator assembly comprising a linkage (136) disposed within the inner lumen and at least one movable actuator pin (132) laterally extending from the linkage (136) and extending through the axially extending slot (134) to releasably couple the actuator assembly to a driver (10) of a robotic surgical instrument holder (4).

2. The surgical instrument (14) of claim 1 wherein the end effector (120) is coupled to the elongate shaft (100) with a wrist.

3. The surgical instrument (14) of claim 1 or 2 wherein axial displacement of the actuator assembly actuates the end effector (120).

4. The surgical instrument (14) of any preceding claim wherein said end effector (120) includes jaws (122,124) that are movable between an open position where the jaws (122,124) are disposed apart and a closed position where the jaws (122,124) are disposed closer together.

5. The surgical instrument (14) of claim 4 wherein the end effector (120) comprises first (122) and second (124) jaws, wherein the first and second jaws (122,124) are both moveable.

6. The surgical instrument (14) of claim 5 wherein the actuator assembly further includes a hinge connecting the linkage (136) to said first and second jaws (122,124) such that axial movement of the at least one actuator pin (132) causes the jaws (122,124) to move between the open and closed positions.

7. The surgical instrument (14) of any of claims 4 to 6 wherein said instrument (14) further comprises means for biasing said jaws (122,124) towards the closed position.

8. The surgical instrument (14) of any preceding claim wherein the elongate shaft (100) includes a pair of opposed axially extending slots (134) and the at least one actuator pin (132) extends laterally through the pair of slots (134).

9. The surgical instrument (14) of any preceding claim wherein the actuator pins (132) are releasably received within an aperture in the driver (80).

10. The surgical instrument (14) of any preceding claim wherein the driver (80) is coupled to the instrument holder (4).

11. The surgical instrument (14) of any preceding claim wherein the actuator pin (132) is releasably received by an aperture of the instrument holder (4).

12. The surgical instrument (14) of any preceding claim wherein the end effector (120) is coupled to the elongate shaft (100) through a plurality of joints,

13. The surgical instrument (14) of claim 1, further comprising:
a mechanical coupling between the proximal and distal ends of said shaft; and
mounting means (116) affixed to and having a protrusion laterally extending from said shaft (100) between the proximal and distal ends for releasably holding the instrument (14);
wherein said actuator assembly is operatively coupled to the end effector (120) for manipulating the end effector (120), said actuator assembly including coupling means for cooperating with an external driver (10) to controllably move the actuator assembly, the coupling means being spaced from and axially movable with respect to the mounting means (116).

14. The instrument (14) of claim 13 wherein said coupling means is axially displaceable, and wherein axial displacement of said coupling means operates the end effector (120).

15. The instrument (14) of claim 13, or 14 wherein said inner lumen extends from said distal end to a point intermediate to said distal and axial ends, and wherein said linkage (136) connects said coupling means with said end effector (120).

16. The surgical instrument (14) of claim 1, wherein
said end effector (120) is movable in a first degree of freedom;
said linkage (136) is connected to the end effector (120) to cause movement in said first degree of freedom in response to movement of said linkage (136);
said at least one pin (132) is coupled to said linkage (136) such that driving said at least one pin (132) causes movement of said linkage (136); and wherein
said shaft (100) is detachably coupleable to an actuating mechanism for selectively driving said at least one pin (132).

17. The instrument (14) of claim 16, further comprising said actuating mechanism.

18. The instrument of claim 16, wherein said movement of said linkage (136) comprises pulling said linkage (136).

19. The instrument (14) of claim 16, wherein said end effector (120) comprising a pair of jaws (122,124), and wherein said at least one pin (132) is axially slidable to open and close said pair of jaws (122,124).

20. The instrument (14) of claim 19, further comprising said actuating mechanism, wherein said actuating mechanism comprises a rotatable actuating body (84) coupleable with said at least one pin (132), said at least one pin (132) driveable by said rotatable actuating body (84) when said at least one pin (132) and actuating body (84) are operably engaged.

21. The instrument of claim 20, wherein said actuating mechanism further comprises a second rotatable actuating body for moving said shaft (100) in a second degree of freedom,

22. The instrument (14) of claim 21, wherein said second rotatable actuating body is operable to rotate said shaft (100) about its longitudinal axis.

23. The instrument (14) of claim 21, wherein said second rotatable actuating body is operable to move said shaft (100) axially along its longitudinal axis.

24. The instrument (14) of claim 16, wherein said end effector (120) comprises a scalpel.

25. A surgical system comprising:
a surgical instrument (14) according to any preceding claim; and
an instrument holder (4) for releasably holding the surgical instrument (14), the instrument holder (4) comprising a body (60), a driver (80), and an instrument support (70) movably mounted on said body (60) and having an interface engageable with the surgical instrument (14) to releasably mount the instrument (14) to said instrument holder (4).

26. The surgical system of claim 25, further comprising a device (300) for moving said instrument about a desired spherical center of rotation (308) at a desired location along said instrument (14), the device (300) comprising:
a base for supporting said instrument holder (4);
a first linkage (321), including at least a first rod, pivotally mounted on said base for rotation about a first axis, said instrument holder (4) holding said instrument (14) in a position so that the desired remote center of spherical rotation (308) along the instrument (14) intersects said first axis; and
a second linkage (323), including at least a second rod, connected to said instrument holder (4) for moving said instrument holder (4) in a parallel-plane relationship to said second linkage (323), said second linkage (323) being pivotally connected to said first linkage (321) so that said second rod remains parallel to said first axis and said first rod remains parallel to the instrument (14), such that said spherical center of rotation (308) is maintained at the desired fixed location.

27. The system of claim 25, further comprising a drive assembly (7) operatively coupled to said instrument holder (4) for providing said instrument (14) with at least two degrees of freedom.

28. The system of claim 27, wherein said drive assembly comprises a first controllable motor (26) operatively connected to move said instrument support (70) and a second controllable motor (30) operatively connected to move said instrument holder body (60) relative to said support base.

29. The system of claim 26 or 27 further comprising a coupling means for removably attaching said instrument holder (4) to said support base and said drive assembly (7).

30. The system of any of claims 25 to 29 wherein said device (300) for moving said instrument about a desired spherical center of rotation (308) is fixable to a surface at an orientation with respect to the vertical, the system further including an inclinometer (350) mounted to said device (300) for measuring said orientation.

31. The system of claim 25, further comprising:
a base (324);
a first elongate member mounted on said base (324), said first elongate member axially rotatable relative to said base about a first longitudinal axis;
a second elongate member coupled to said first elongate member, wherein axial rotation of said first elongate member about said first longitudinal axis causes corresponding rotation of said second elongate member about said first longitudinal axis, said second elongate member coupled to said instrument holder;
wherein the longitudinal axis of said elongate shaft and said first longitudinal axis intersect at a remote center of rotation (308) lying on said elongate shaft.

32. A method for controlling a surgical instrument comprising:
providing a surgical instrument (14) removably coupleable to an actuating mechanism (80), said instrument (14) comprising proximal and distal portions, said proximal portion comprising at least one movable actuator pin (132) engageable with an actuating body (84) on said actuating mechanism (80), said instrument (14) including at least one distal joint coupled to an end effector member (120), said actuated body (84) coupled to said distal joint by at least one elongate element (136), said elongate element (136) being housed in the inner lumen of a shaft portion (100) of said instrument (14) and extending between said proximal and distal portions, said shaft portion having at least one axially extending slot (134), said at least one movable pin (132) laterally extending from said elongate element (136) and extending through the axially extending slot;
coupling said instrument (14) to said actuating mechanism (80) by engaging said at least one movable actuator pin (132) with said actuating mechanism (80); and
controlling the operation of said actuating mechanism (80) from a remote location so that said actuating body (84) on said actuating mechanism (80) drives said at least one movable actuator pin (132) of the instrument (14), thereby causing the at least one distal joint of the instrument (14) to move,

## Patentansprüche

1. Chirurgisches Instrument (14), das umfasst:
einen lang gestreckten Schaft (100) mit einem proximalen Abschnitt, einem distalen Abschnitt, einem inneren Lumen und wenigstens einem axial verlaufenden Schlitz (134);
einen Effektor (120), der in der Nähe des distalen Abschnitts des lang gestreckten Schafts (100) angeordnet ist; und
eine Aktoranordnung, die ein in dem inneren Lumen angeordnetes Verbindungsglied (136) und wenigstens einen beweglichen Aktorstift (132), der von dem Verbindungsglied (136) seitlich absteht und sich durch den axial verlaufenden Schlitz (134) erstreckt, um die Aktoranordnung mit einem Antrieb (10) einer Halterung (4) für ein robotisches chirurgisches Instrument lösbar zu koppeln.

2. Chirurgisches Instrument (14) nach Anspruch 1, wobei der Effektor (120) mit dem lang gestreckten Schaft (100) über ein Gelenk gekoppelt ist.

3. Chirurgisches Instrument (14) nach Anspruch 1 oder 2, wobei die axiale Verlagerung der Aktoranordnung den Effektor (120) betätigt

4. Chirurgisches Instrument (14) nach einem vorhergehenden Anspruch, wobei der Effektor (120) Klemmbacken (122, 124) enthält, die zwischen einer geöffneten Position, in der die Klemmbacken (122, 124) voneinander beabstandet angeordnet sind, und einer geschlossenen Position, in der die Klemmbacken (122, 124) näher beieinander angeordnet sind, beweglich sind.

5. Chirurgisches Instrument (14) nach Anspruch 4, wobei der Effektor (120) eine erste Klemmbacke (122) und eine zweite Klemmbacke (124) umfasst, wobei sowohl die erste als auch die zweite Klemmbacke (122, 124) beweglich sind.

6. Chirurgisches Instrument (14) nach Anspruch 5, wobei die Aktoranordnung ferner ein Scharnier enthält, das das Verbindungsglied (136) mit der ersten und der zweiten Klemmbacke ('122, 124) verbindet, derart, dass eine axiale Bewegung des wenigstens einen Aktorstifts (132) die Klemmbacken (122, 124) dazu veranlasst, sich zwischen der geöffneten und der geschlossenen Position zu bewegen.

7. Chirurgisches Instrument (14) nach einem der Ansprüche 4 bis 6, wobei das Instrument (14) ferner Mittel zum Vorbelasten der Klemmbacken (122, 124) in die geschlossene Position umfasst.

8. Chirurgisches Instrument (14) nach einem vorhergehenden Anspruch, wobei der lang gestreckte Schaft (100) ein Paar sich axial entgegengesetzt erstreckender Schlitze (134) aufweist und der wenigstens eine Aktorstift (132) sich seitlich durch das Schlitzpaar (134) erstreckt.

9. Chirurgisches Instrument (14) nach einem vorhergehenden Anspruch, wobei die Aktorstifte (132) in einer Öffnung in dem Antrieb (80) lösbar aufgenommen sind.

10. Chirurgisches Instrument (14) nach einem vorhergehenden Anspruch, wobei der Antrieb (80) mit dem Instrumentenhalter (4) gekoppelt ist.

11. Chirurgisches Instrument (14) nach einem vorhergehenden Anspruch, wobei der Aktorstift (132) durch eine Öffnung des Instrumentenhalters (4) lösbar aufgenommen ist.

12. Chirurgisches Instrument (14) nach einem vorhergehenden Anspruch, wobei der Effektor (120) mit dem lang gestreckten Schaft (100) über mehrere Gelenke verbunden ist.

13. Chirurgisches Instrument (14) nach Anspruch 1, das ferner umfasst:
eine mechanische Kopplung zwischen dem proximalen und dem distalen Ende des Schafts, und
Anbringungsmittel (116), die an dem Schaft (100) zwischen dem proximalen und dem distalen Ende befestigt sind und einen hiervon sich axial erstreckenden Vorsprung aufweisen, um das Instrument (14) lösbar zu halten;
wobei die Aktoranordnung mit dem Effektor (120) funktional gekoppelt ist, um den Effektor (120) zu manipulieren, wobei die Aktoranordnung Kopplungsmittel enthält, um mit einem äußeren Antrieb (10) zusammenzuwirken, um die Aktoranordnung steuerbar zu bewegen, wobei die Kopplungsmittel von den Anbringungsmitteln (116) beabstandet und in Bezug hierzu axial beweglich sind.

14. Instrument (14) nach Anspruch 13, wobei die Kopplungsmittel axial verlagerbar sind und wobei die axiale Verlagerung der Kopplungsmittel den Effektor (120) betreibt.

15. Instrument (14) nach Anspruch 13 oder 14, wobei sich das innere Lumen von dem distalen Ende zu einem Punkt zwischen dem distalen und dem axialen Ende erstreckt und wobei das Verbindungsglied (136) die Kopplungsmittel mit dem Effektor (120) verbindet.

16. Chirurgisches Instrument (14) nach Anspruch 1, wobei
der Effektor (120) in einem ersten Freiheitsgrad beweglich ist;
das Verbindungsglied (136) mit dem Effektor (120) verbunden ist, um in Reaktion auf die Bewegung des Verbindungsgliedes (136) eine Bewegung in dem ersten Freiheitsgrad zu verursachen;
der wenigstens eine Stift (132) mit dem Verbindungsglied (136) gekoppelt ist, so dass der Antrieb des wenigstens einen Stifts (132) eine Bewegung des Verbindungsglieds (136) verursacht; und wobei
der Schaft (100) mit einem Betätigungsmechanismus abnehmbar gekoppelt werden kann, um den wenigstens einen Stift (132) wahlweise anzutreiben.

17. Instrument (14) nach Anspruch 16, das ferner den Betätigungsmechanismus umfasst.

18. Instrument nach Anspruch 16, wobei die Bewegung des Verbindungsgliedes (136) das Ziehen des Verbindungsgliedes (136) umfasst.

19. Instrument (14) nach Anspruch 16, wobei der Effektor (120) ein Paar Klemmbacken (122, 124) umfasst und wobei der wenigstens eine Stift (132) axial gleiten kann, um das Paar Klemmbacken (122, 124) zu öffnen und zu schließen,

20. Instrument (14) nach Anspruch 19, das ferner den Betätigungsmechanismus umfasst, wobei der Betätigungsmechanismus einen drehbaren Betätigungskörper (84) umfasst, der mit dem wenigstens einen Stift (132) koppelbar ist, wobei der wenigstens eine Stift (132) durch den drehbaren Betätigungskörper (84) angetrieben werden kann, wenn der wenigstens eine Stift (132) und der BetätigungsiCörper (84) in einem funktionalen Eingriff sind.

21. Instrument nach Anspruch 20, wobei der Betätigungsmechanismus ferner einen zweiten drehbaren Betätigungskörper umfasst, um den Schaft (100) in einem zweiten Freiheitsgrad zu bewegen.

22. Instrument (14) nach Anspruch 21, wobei der zweite drehbare Betätigungskörper betreibbar ist, um den Schaft (100) um seine Längsachse zu drehen.

23. Instrument (14) nach Anspruch 21, wobei der zweite drehbare Betätigungskörper betreibbar ist, um den Schaft (100) axial entlang seiner Längsachse zu bewegen

24. Instrument (14) nach Anspruch 16, wobei der Effektor (120) ein Skalpell umfasst.

25. Chirurgisches System, das umfasst:
ein chirurgisches Instrument (14) nach einem vorhergehenden Anspruch; und
einen Instrumentenhalter (4), um das chirurgische Instrument (14) lösbar zu halten, wobei der Instrumentenhalter (4) einen Körper (60), einen Antrieb (80) und einen Instrumententräger (70), der an dem Körper (60) beweglich angebracht ist und eine Schnittstelle besitzt, die mit dem chirurgischen Instrument (14) in Eingriff gelangen kann, um das Instrument (14) an dem Instrumentenhalter (4) lösbar anzubringen, umfasst.

26. Chirurgisches System nach Anspruch 25, das ferner eine Vorrichtung (300) zum Bewegen des Instruments um ein gewünschtes sphärisches Drehzentrum (308) an einem gewünschten Ort längs des Instruments (14) umfasst, wobei die Vorrichtung (300) umfasst:
eine Basis, um den Instrumentenhalter (4) zu unterstützen;
ein erstes Verbindungsglied (321), das einen ersten Stab, der an der Basis schwenkbar angebracht ist, um sich um eine erste Achse zu drehen, enthält, wobei der Instrumentenhalter (4) das Instrument (14) an einer Position hält, so dass das gewünschte entfernte sphärische Drehzentrum (308) längs des Instruments (14) die erste Achse schneidet; und
ein zweites Verbindungsglied (323), das einen zweiten Stab, der mit dem Instrumentenhalter (4) verbunden ist, um den Instrumentenhalter (4) in einer planparallelen Beziehung zu dem zweiten Verbindungsglied (323) zu bewegen, enthält, wobei das zweite Verbindungsglied (323) mit dem ersten Verbindungsglied (321) schwenkbar verbunden ist, so dass der zweite Stab zu der ersten Achse parallel bleibt und der erste Stab zu dem Instrument (14) parallel bleibt, derart, dass das sphärische Drehzentrum (308) an dem gewünschten festen Ort gehalten wird.

27. System nach Anspruch 25, das ferner eine Antriebsanordnung (7) umfasst, die mit dem Instrumentenhalter (4) funktional gekoppelt ist, um das Instrument (14) mit wenigstens zwei Freiheitsgraden zu versehen.

28. System nach Anspruch 27, wobei die Antriebsanordnung einen ersten steuerbaren Motor (26), der funktional so verbunden ist, dass er den Instrumententräger (70) bewegt, und einen zweiten steuerbaren Motor (30), der funktional so verbunden ist, dass er den Instrumentenhalterkörper (60) in Bezug auf die Unterstützungsbasis bewegt, umfasst.

29. System nach Anspruch 26 oder 27, das ferner ein Kopplungsmittel umfasst, um den Instrumentenhalter (4) an der Unterstützungsbasis und an der Antriebsanordnung (7) lösbar zu befestigen.

30. System nach einem der Ansprüche 25 bis 29, wobei die Vorrichtung (300) zum Bewegen des Instruments um ein gewünschtes sphärisches Drehzentrum (308) an einer Oberfläche mit einer Orientierung in Bezug auf die Vertikale befestigbar ist,
wobei das System ferner einen Neigungsmesser (350) aufweist, der an der Vorrichtung (300) angebracht ist, um die Orientierung zu messen.

31. System nach Anspruch 25, das ferner umfasst:
eine Basis (324);
ein erstes lang gestrecktes Element, das an der Basis (324) angebracht ist,
wobei das erste lang gestreckte Element in Bezug auf die Basis um eine erste Längsachse axial drehbar ist;
ein zweites lang gestrecktes Element, das mit dem ersten lang gestreckten Element gekoppelt ist, wobei die axiale Drehung des ersten lang gestreckten Elements um die erste Längsachse eine entsprechende Drehung des zweiten lang gestreckten Elements um die erste Längsachse zur Folge hat, wobei das zweite lang gestreckte Element mit dem Instrumentenhalter gekoppelt ist;
wobei sich die Längsachse des lang gestreckten Schafts und die erste Längsachse an einem entfernten Drehzentrum (308), das auf dem lang gestreckten Schaft liegt, schneiden.

32. Verfahren zum Steuern eines chirurgischen Instruments, das umfasst:
Vorsehen eines chirurgischen Instruments (14), das mit einem Betätigungsmechanismus (80) abnehmbar koppelbar ist, wobei das Instrument (14) einen proximalen und einen distalen Abschnitt umfasst, wobei der proximale Abschnitt wenigstens einen beweglichen Aktorstift (132) aufweist, der mit einem Aktorkörper (84) an dem Betätigungsmechanismus (80) in Eingriff gelangen kann, wobei das Instrument (14) wenigstens ein distales Gelenk, das mit einem Effektor-Element (120) gekoppelt ist, aufweist, der Betätigungskörper (84) mit dem distalen Gelenk durch wenigstens ein lang gestrecktes Element (136) gekoppelt ist, das lang gestreckte Element (136) in dem inneren Lumen eines Schaftabschnitts (100) des Instruments (14) untergebracht ist und sich zwischen dem proximalen und dem distalen Abschnitt erstreckt, der Schaftabschnitt wenigstens einen axial verlaufenden Schlitz (134) aufweist und der wenigstens eine bewegliche Stift (132) von dem lang gestreckten Element (136) absteht und sich durch den axial verlaufenden Schlitz erstreckt;
Koppeln des Instruments (14) mit dem Betätigungsmechanismus (80) durch Ergreifen des wenigstens einen beweglichen Aktorstifts (132) mit dem Betätigungsmechanismus (80); und
Steuern des Betriebs des Betätigungsmechanismus (80) von einem entfernten Ort aus, so dass der Betätigungskörper (84) an dem Betätigungsmechanismus (80) den wenigstens einen beweglichen Aktorstift (132) des Instruments (14) antreibt, um dadurch das wenigstens eine distale Gelenk des Instruments (14) zu einer Bewegung zu veranlassen.

## Revendications

1. Instrument chirurgical (14) comprenant :
une tige allongée (100) dotée d'une partie proximale, d'une partie distale, d'une lumière interne et d'au moins une fente s'étendant axialement (134) ;
un effecteur d'extrémité (120) disposé de manière adjacente par rapport à la partie distale de la tige allongée (100) ; et
un ensemble d'actionneur comprenant une liaison (136) disposée à l'intérieur de la lumière interne et au moins un axe d'actionneur mobile (132) s'étendant latéralement à partir de la liaison (136) et s'étendant à travers la fente s'étendant axialement (134) de manière à coupler de façon libérable l'ensemble d'actionneur à un dispositif d'entraînement (10) d'un support d'instrument chirurgical robotique (4).

2. Instrument chirurgical (14) selon la revendication 1, dans lequel l'effecteur d'extrémité (120) est couplé à la tige allongée (100) au moyen d'un poignet.

3. Instrument chirurgical (14) selon la revendication 1 ou la revendication 2, dans lequel le déplacement axial de l'ensemble d'actionneur actionne l'effecteur d'extrémité (120).

4. Instrument chirurgical (14) selon l'une quelconque des revendications précédentes, dans lequel ledit effecteur d'extrémité (120) inclut des mâchoires (122, 124) qui sont mobiles entre une position ouverte où les mâchoires (122, 124) sont éloignées les unes des autres et une position fermée où les mâchoires (122, 124) sont disposées de façon plus proche les unes des autres.

5. Instrument chirurgical (14) selon la revendication 4, dans lequel l'effecteur d'extrémité (120) comprend des première (122) et seconde (124) mâchoires, lesquelles première et seconde mâchoires (122, 124) sont toutes deux mobiles.

6. Instrument chirurgical (14) selon la revendication 5, dans lequel l'ensemble d'actionneur inclut en outre une articulation reliant la liaison (136) auxdites première et seconde mâchoires (122, 124) de manière à ce que le mouvement axial du au moins un axe d'actionneur (132) fasse en sorte que les mâchoires (122, 124) se déplacent entre la position ouverte et la position fermée.

7. Instrument chirurgical (14) selon l'une quelconque des revendications 4 à 6, dans lequel ledit instrument (14) comprend en outre des moyens destinés à influencer lesdites mâchoires (122, 124) vers la position fermée.

8. Instrument chirurgical (14) selon l'une quelconque des revendications précédentes, dans lequel la tige allongée (100) inclut une paire de fentes s'étendant axialement (134) opposées et le au moins un axe d'actionneur (132) s'étend latéralement à travers la paire de fentes (134).

9. Instrument chirurgical (14) selon l'une quelconque des revendications précédentes, dans lequel les axes d'actionneur (132) sont reçus de façon libérable à l'intérieur d'une ouverture dans le dispositif d'entraînement (80).

10. Instrument chirurgical (14) selon l'une quelconque des revendications précédentes, dans lequel le dispositif d'entraînement (80) est couplé au support d'instrument (4).

11. Instrument chirurgical (14) selon l'une quelconque des revendications précédentes, dans lequel l'axe d'actionneur (132) est reçu de façon libérable par une ouverture du support d'instrument (4).

12. Instrument chirurgical (14) selon l'une quelconque des revendications précédentes, dans lequel l'effecteur d'extrémité (120) est couplé à la tige allongée (100) au moyen d'une pluralité de jonctions.

13. Instrument chirurgical (14) selon la revendication 1, comprenant en outre :
un couplage mécanique entre les extrémités proximale et distale de ladite tige; et
des moyens de montage (116) fixés à et ayant une protubérance s'étendant latéralement à partir de ladite tige (100) entre les extrémités proximale et distale en vue de supporter de façon libérable l'instrument (14) ;
dans lequel ledit ensemble d'actionneur est couplé de façon opérationnelle à l'effecteur d'extrémité (120) afin de manipuler l'effecteur d'extrémité (120), ledit ensemble d'actionneur incluant des moyens de couplage destinés à coopérer avec un dispositif d'entraînement extérieur (10) en vue de déplacer de façon contrôlée l'ensemble d'actionneur, les moyens de couplage étant espacés des moyens de montage (116) et pouvant se déplacer axialement par rapport à ces derniers.

14. Instrument (14) selon la revendication 13, dans lequel lesdits moyens de couplage peuvent être déplacés axialement, et dans lequel le déplacement axial desdits moyens de couplage fait fonctionner l'effecteur d'extrémité (120).

15. Instrument (14) selon la revendication 13 ou la revendication 14, dans lequel ladite lumière interne s'étend à partir de ladite extrémité distale jusqu'à un point intermédiaire par rapport auxdites extrémités distale et axiale, et dans lequel ladite liaison (136) relie lesdits moyens de couplage et ledit effecteur d'extrémité (120).

16. Instrument chirurgical (14) selon la revendication 1, dans lequel
ledit effecteur d'extrémité (120) est mobile suivant un premier degré de liberté ;
ladite liaison (136) est reliée à l'effecteur d'extrémité (120) en vue d'entraîner le mouvement suivant ledit premier degré de liberté en réponse au mouvement de ladite liaison (136) ;
ledit au moins un axe (132) est couplé à ladite liaison (136) de manière à ce que l'entraînement dudit au moins un axe (132) entraîne le mouvement de ladite liaison (136) ; et dans lequel
ladite tige (100) peut être couplée de façon détachable à un mécanisme d'actionnement de manière à entraîner de façon sélective ledit au moins un axe (132).

17. Instrument (14) selon la revendication 16, comprenant en outre ledit mécanisme d'actionnement.

18. Instrument selon la revendication 16, dans lequel ledit mouvement de ladite liaison (136) comprend la traction de ladite liaison (136).

19. Instrument (14) selon la revendication 16, dans lequel ledit effecteur d'extrémité (120) comprend une paire de mâchoires (122, 124), et dans lequel ledit au moins un axe (132) peut coulisser axialement de manière à ouvrir et fermer ladite paire de mâchoires (122, 124).

20. Instrument (14) selon la revendication 19, comprenant en outre ledit mécanisme d'actionnement, dans lequel ledit mécanisme d'actionnement comprend un corps d'actionnement rotatif (84) pouvant être couplé audit au moins un axe (132), ledit au moins un axe (132) pouvant être entraîné par ledit corps d'actionnement rotatif (84) lorsque ledit au moins un axe (132) et ledit corps d'actionnement (84) sont mis en prise de façon opérationnelle.

21. Instrument selon la revendication 20, dans lequel ledit mécanisme d'actionnement comprend en outre un second corps d'actionnement rotatif destiné à déplacer ladite tige (100) suivant un second degré de liberté.

22. Instrument (14) selon la revendication 21, dans lequel ledit second corps d'actionnement rotatif peut être actionné de manière à faire tourner ladite tige (100) autour de son axe longitudinal.

23. Instrument (14) selon la revendication 21, dans lequel ledit second corps d'actionnement rotatif peut être actionné de manière à déplacer ladite tige (100) axialement le long de son axe longitudinal.

24. Instrument (14) selon la revendication 16, dans lequel ledit effecteur d'extrémité (120) comprend un scalpel.

25. Système chirurgical comprenant :
un instrument chirurgical (14) selon l'une quelconque des revendications précédentes ; et
un support d'instrument (4) destiné à supporter de façon libérable l'instrument chirurgical (14), le support d'instrument (4) comprenant un corps (60), un dispositif d'entraînement (80) et un support d'instrument (70) monté de façon mobile sur ledit corps (60) et ayant une interface apte à venir en prise avec l'instrument chirurgical (14) pour monter de façon libérable l'instrument (14) sur ledit support d'instrument (4).

26. Système chirurgical selon la revendication 25, comprenant en outre un dispositif (300) destiné à déplacer ledit instrument autour d'un centre de rotation sphérique souhaité (308) à un emplacement souhaité le long dudit instrument (14), le dispositif (300) comprenant :
une embase destinée à supporter ledit support d'instrument (4) ;
une première liaison (321), incluant au moins une première tige, montée de façon pivotante sur ladite embase destinée à tourner autour d'un premier axe, ledit support d'instrument (4) supportant ledit instrument (14) dans une position de manière à ce que le centre de rotation sphérique distant souhaité (308) le long de l'instrument (14) croise ledit premier axe ; et
une seconde liaison (323), incluant au moins une seconde tige, reliée audit support d'instrument (4) destiné à déplacer ledit support d'instrument (4) de façon parallèle au plan par rapport à ladite seconde liaison (323), ladite seconde liaison (323) étant reliée de façon pivotante à ladite première liaison (321) de manière à ce que ladite seconde tige reste parallèle audit premier axe et de manière à ce que ladite première tige reste parallèle à l'instrument (14), de telle sorte que ledit centre de rotation sphérique (308) est maintenu à l'emplacement fixé souhaité.

27. Système selon la revendication 25, comprenant en outre un ensemble d'entraînement (7) couplé de façon opérationnelle audit support d'instrument (4) afin de fournir audit instrument (14) au moins deux degrés de liberté.

28. Système selon la revendication 27, dans lequel ledit ensemble d'entraînement comprend un premier moteur contrôlable (26) relié de façon opérationnelle pour déplacer ledit support d'instrument (70) et un second moteur contrôlable (30) relié de façon opérationnelle pour déplacer ledit corps du support d'instrument (60) par rapport à ladite embase de support.

29. Système selon la revendication 26 ou la revendication 27, comprenant en outre des moyens de couplage destinés à attacher de façon amovible ledit support d'instrument (4) à ladite embase de support et audit ensemble d'entraînement (7).

30. Système selon l'une quelconque des revendications 25 à 29, dans lequel ledit dispositif (300) destiné à déplacer ledit instrument autour d'un centre de rotation sphérique souhaité (308) peut être fixé sur une surface suivant une orientation par rapport à la verticale, le système incluant en outre un inclinomètre (350) monté sur ledit dispositif (300) destiné à mesurer ladite orientation.

31. Système selon la revendication 25, comprenant en outre :
une embase (324) ;
un premier élément allongé monté sur ladite embase (324), ledit premier élément allongé pouvant tourner axialement par rapport à ladite embase autour d'un premier axe longitudinal ;
un second élément allongé couplé audit premier élément allongé, dans lequel la rotation axiale dudit premier élément allongé autour dudit premier axe longitudinal entraîne une rotation correspondante dudit second élément allongé autour dudit premier axe longitudinal, ledit second élément allongé étant couplé audit support d'instrument ;
dans lequel l'axe longitudinal de ladite tige allongée et ledit premier axe longitudinal se croisent à un centre de rotation distant (308) reposant sur ladite tige allongée.

32. Procédé permettant de contrôler un instrument chirurgical comprenant :
la fourniture d'un instrument chirurgical (14) pouvant être couplé de façon amovible à un mécanisme d'actionnement (80), ledit instrument (14) comprenant des parties proximale et distale, ladite partie proximale comprenant au moins un axe d'actionneur mobile (132) pouvant être mis en prise avec un corps d'actionnement (84) sur ledit mécanisme d'actionnement (80), ledit instrument (14) incluant au moins une jonction distale couplée à un élément d'effecteur d'extrémité (120), ledit corps actionné (84) étant couplé à ladite jonction distale au moyen d'au moins un élément allongé (136), ledit élément allongé (136) étant logé dans la lumière interne d'une partie de tige (100) dudit instrument (14) et s'étendant entre lesdites parties proximale et distale, ladite partie de tige ayant au moins une fente s'étendant axialement (134), ledit au moins un axe mobile (132) s'étendant latéralement à partir dudit élément allongé (136) et s'étendant à travers la fente s'étendant axialement ;
le couplage dudit instrument (14) audit mécanisme d'actionnement (80) en mettant en prise ledit au moins un axe d'actionneur mobile (132) avec ledit mécanisme d'actionnement (80) ; et
le contrôle du fonctionnement dudit mécanisme d'actionnement (80) à partir d'un emplacement distant de telle sorte que ledit corps d'actionnement (84) sur ledit mécanisme d'actionnement (80) entraîne ledit au moins un axe d'actionneur mobile (132) de l'instrument (14), entraînant ainsi le mouvement du au moins une jonction distale de l'instrument (14).
